(19) Europäisches Patentamt

European Patent Office

Office européen des brevets

(11) **EP 1 022 984 B1**

(12) **EUROPÄISCHE PATENTSCHRIFT**

(45) Veröffentlichungstag und Bekanntmachung des Hinweises auf die Patenterteilung:
**14.04.2004 Patentblatt 2004/16**

(51) Int Cl.$^7$: **A61B 5/0275**, A61B 5/00, A61B 5/026

(21) Anmeldenummer: **99942720.6**

(22) Anmeldetag: **01.07.1999**

(86) Internationale Anmeldenummer:
**PCT/DE1999/001899**

(87) Internationale Veröffentlichungsnummer:
**WO 2000/001297 (13.01.2000 Gazette 2000/02)**

(54) **BESTIMMUNG DER LEBERFUNKTION ANHAND EINER PLASMAVERSCHWINDERATE**

DETERMINATION OF LIVER FUNCTION USING THE RATE AT WHICH PLASMA DISAPPEARS

DETERMINATION DE LA FONCTION HEPATIQUE SUR LA BASE D'UNE VITESSE DE DISPARITION DE PLASMA

(84) Benannte Vertragsstaaten:
**DE ES FR GB IT**

(30) Priorität: **01.07.1998 DE 19829273**

(43) Veröffentlichungstag der Anmeldung:
**02.08.2000 Patentblatt 2000/31**

(73) Patentinhaber: **Hoeft, Andreas, Prof. Dr. med.
53127 Bonn (DE)**

(72) Erfinder: **Hoeft, Andreas, Prof. Dr. med.
53127 Bonn (DE)**

(74) Vertreter: **Kehl, Günther, Dipl.-Phys.
Patentanwaltskanzlei
Günther Kehl
Friedrich-Herschel-Strasse 9
81679 München (DE)**

(56) Entgegenhaltungen:
EP-A- 0 359 206        EP-A- 0 399 482
EP-A- 0 403 683        DE-A- 4 130 931

EP 1 022 984 B1

**Beschreibung**

Die Erfindung betrifft eine Vorrichtung zur Bestimmung der Leberfunktion anhand einer Plasmaverschwinderate nach dem Oberbegriff des Anspruchs 1. Eine solche Vorrichtung ist aus Dokument EP-A-0 359 206 bekannt.

Die Leberfunktion ist ein wichtiger Parameter in der Intensivmedizin und für die Prognose von schwerstkranken Patienten entscheidend. Zur Zeit wird die Leberfunktion anhand von verschiedenen Laborgrößen, die die synthetische Leistung und die Eliminationsleistung der Leber charakterisieren, in der Routine in der Intensivmedizin erfaßt. Der Nachteil dieser Laborgrößen ist jedoch, daß sie bei Ausfall der Leberfunktion erst mit einer erheblichen zeitlichen Latenz auf krankhafte Werte verändert sind, so daß eine Leberfunktionsstörung erst nach Tagen evident wird.

Eine Möglichkeit die Leberfunktion, zumindest bezüglich ihrer Eliminationsleistung sofort zu evaluieren, besteht in der Applikation von Indikatorstoffen, die durch die Leber eliminiert werden und der Bestimmung der Eliminationszeitkonstante dieser Indikatoren. Ein hierfür gängiger Indikator ist der Farbstoff Indocyaningrün. Üblicherweise wird das Indocyaningrün als Bolus intravenös injiziert und im Anschluß an die Bolusinjektion wird in Abständen von mehreren Minuten über eine Zeitspanne von mindestens 15 Minuten mindestens 2, meistens mehrere Blutproben entnommen. Aus dem Abfall der Farbstoffkonzentration in den Blutproben kann die Eliminationszeitkonstante berechnet werden. Dieses Verfahren wird in der Klinik jedoch selten angewendet, da es wegen der Laboranalysen immer noch zu zeitaufwendig ist.

Der Erfindung liegt die Aufgabe zugrunde, eine Vorrichtung zu schaffen, mit der die Leberfunktion nichtinvasiv bestimmt werden kann, und das Meßergebnis schneller zur Verfügung steht.

Diese Aufgabe wird bei einer Vorrichtung nach dem Oberbegriff des Anspruchs 1 durch die Merkmale jenes Anspruchs gelöst.

Auch bei der erfindungsgemäßen Vorrichtung wird eine Abnahme einer Farbstoffkonzentration im Blut gemessen, die durch Abbau des Farbstoffes durch die Leber zustandekommt. Nach Injektion eines geeigneten Farbstoffes, wie z. B. Indocyaningrün, in die Blutbahn ergibt sich bei normaler Leberfunktion an einer Meßstelle des Körpers ein charakteristischer Farbstoff-Konzentrations-Zeit-Verlauf. Hier zeigt sich zunächst ein erstes Maximum der Farbstoff-Konzentration und nach einem vorübergehenden Abklingen ein zweites Maximum der Farbstoff-Konzentration. Das zweite Maximum entsteht durch Rezirkulation, d.h. vor Abklingen der Konzentrationswelle bei einem ersten Durchlauf erfolgt bereits ein zweiter Durchlauf.

Aus der Messung des zeitlichen Verlaufs der Farbstoffkonzentration c(t) wird eine mittlere Kreislauftransitzeit $mtt_{circ}$ berechnet und außerdem ein Parameter k ermittelt, der eine fraktionelle Wiedereintrittsrate des Indikatorfarbstoffes nach jeder Kreislaufpassage darstellt. Mit diesen Werten kann dann die Plasmaverschwinderate (PDR) nach der Formel

$$PDR = (1\text{-}k) \, / \, (k \cdot mtt_{circ})$$

berechnet werden. Das Ergebnis liegt bereits nach wenigen Rezirkulationszyklen vor.

Die zuvor angegebene mittlere Kreislauftransitzeit $mtt_{circ}$ kann aus einer Kreislauftransportfunktion g(t) berechnet werden, die das Transportverhalten des Kreislaufs beschreibt. Die mittlere Kreislauftransitzeit $mtt_{circ}$ wird dann nach der Formel

$$mtt_{circ} = \frac{\int\limits_{0}^{\infty} g(t) \cdot t \, dt}{\int\limits_{0}^{\infty} g(t) \, dt}$$

erhalten.

Die Berechnung der Kreislauftransportfunktion g(t) kann aus der gemessenen Farbstoffkonzentration mit Hilfe eines iterativen, nichtlinearen Anpassungsverfahrens durchgeführt werden. Hier wird unter Vorgabe einer Modellfunktion

$$g(t) = a_m g_m(t) + a_{m+1} g_{m+1}(t) + \dots + a_n g_n(t)$$

mit

$$\sum_{m=1}^{n} a_m = 1,$$

bei der die einzelnen Kompartimente amgm durch linksschiefe Verteilungsfunktionen beschrieben sind, wiederholt eine rekursive Faltung gemäß den Gleichungen

$$c(t) = c_{bolus}(t) + c_{rez}(t)$$

und

$$c_{rez}(t) = k * \int_0^t g(t-u) * c(u) \, du$$

durchgeführt. Dabei werden die Parameter k, $a_m$ sowie die Parameter der Verteilungsfunktionen nach dem Verfahren der kleinsten quadratischen Abweichung optimiert, wobei wenigstens ein Kompartiment $a_1g_1(t)$ angesetzt wird. In der Formel bilden c(t) den Konzentrations-Zeit-Verlauf des Indikatorfarbstoffes, $c_{bolus}(t)$ der den Meßort direkt passierende erste Anteil des Farbstoff-Konzentrations-Zeit-Verlaufs, $c_{rez}(t)$ einen rezirkulierender Anteil des Farbstoff-Konzentrations-Zeit-Verlaufs und k die Eliminationsfraktion des durch die Leber eliminierten Farbstoffes.

Für eine größere Genauigkeit können auch zwei Kompartimente $(a_1g_1(t))$ und $(a_2g_2(t))$ angesetzt werden.

Die optische Messung der im Blutkreislauf resultierenden Farbstoffkonzentration kann alternativ durch fiberoptische Messung in einem zentralen Gefäß vorgenommen werden oder nicht-invasiv mittels Licht-Transmissions- oder Reflexionsmessung eingestrahlten Lichtes an geeigneten Körperorten, insbesondere am Finger, Ohrläppchen, Nasenrücken, Wangenschleimhaut oder Stirn.

Nachfolgend wird die Erfindung anhand der Zeichnung erläutert. In dieser zeigen:

Fig. 1     eine grafische Darstellung des Konzentrations-Zeit-Verlaufs eines Indikatorfarbstoffes,

Fig. 2     eine halblogarithmische Darstellung des Konzentrations-Zeit-Verlaufs nach Fig. 1,

Fig. 3     eine grafische Darstellung der Kreislauftransportfunktion und

Fig. 4     ein Blockschaltbild einer Vorrichtung zur Messung und Bestimmung der Plasmaverschwinderate

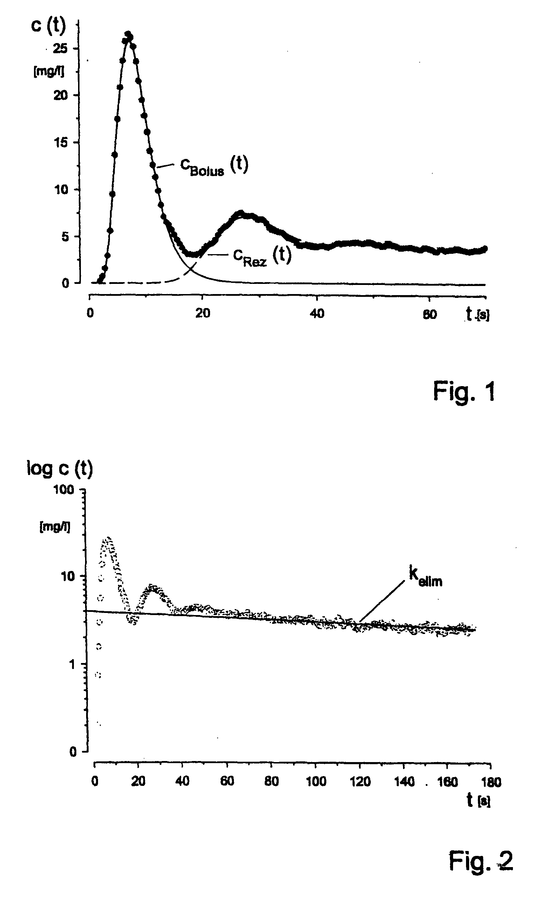

Fig. 1 zeigt eine grafische Darstellung des Konzentrations-Zeit-Verlaufs eines Indikatorfarbstoffes nach einer Bolusinjektion.

Das nach dem ersten Maximum folgende zweite Maximum entsteht durch Rezirkulation, d.h. vor Abklingen der Konzientrationswelle bei einem ersten Durchlauf erfolgt bereits ein zweiter Durchlauf.

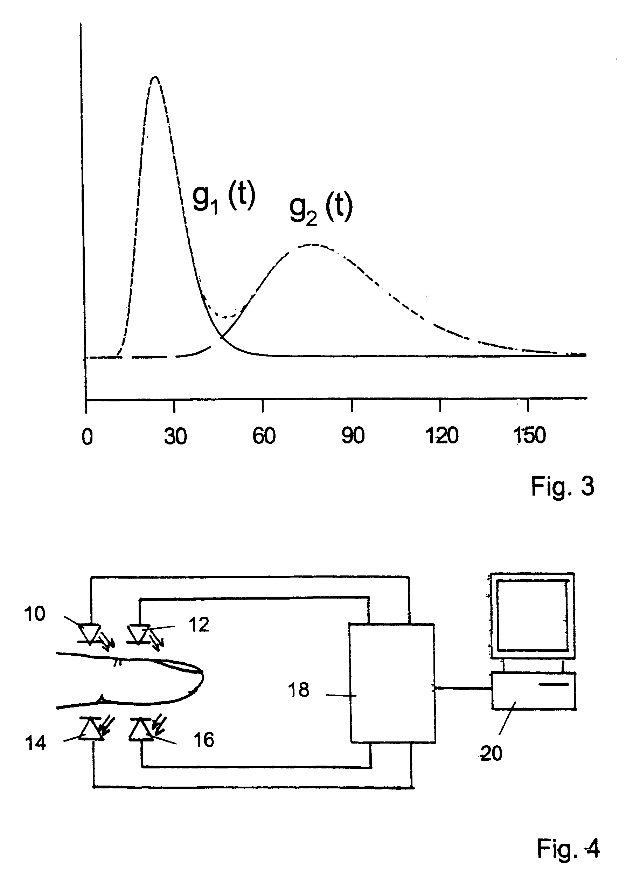

An einer Meßstelle des Körpers sind zur Erfassung des Farbstoff-Konzentrations-Zeit-Verlaufs Sensoren angeordnet, die mit einer Auswerteschaltung und einem Rechner verbunden sind. Ein Beispiel für eine erfindungsgemäße Vorrichtung zeigt Fig. 4. Dort sind zwei Lichtsender 10 und 12 und zwei Lichtempfänger 14 und 16 vorgesehen, die über eine Auswerteschaltung 18 mit einem Rechner 20 verbunden sind. Es sei davon ausgegangen, daß für die Messung der Leberfunktion als Indikatorfarbstoff Indocyaningrün verwendet wird.

Die Verwendung eines Referenzfarbstoffes ist günstig aber nicht zwingend notwendig. Als Referenzfarbstoff kann der rote Blutfarbstoff (Hämoglobin) verwendet werden. In diesem Fall arbeitet der eine Lichtsender 10 auf einer Wellenlänge von 800 Nanometern und der andere Lichtsender 12 auf einer Wellenlänge von 900 Nanometern. Die Lichtempfänger 14 und 16 können so ausgeführt sein, daß sie auch bevorzugt diese Wellenlängen herausfiltern. Alternativ kann aber auch zwischen zwei Lichtsendern 10 und 12 umgeschaltet werden und man kommt in diesem Fall mit nur einem Lichtempfänger 14 aus, der aber für beide Wellenlängen ausgelegt sein muß.

Die Messung des Absorptionsverhaltens der Farbstoffe kann sowohl durch Licht-Transmissions- oder auch durch Licht-Reflexionsmessung erfolgen. Geeignete Körperorte sind die Finger, die Ohrläppchen, der Nasenrücken, die Wangenschleimhaut oder die Stirn.

Die von den Lichtempfängern 14 und 16 empfangenen Signale der Lichtintensitäten werden von der Auswerteschaltung 18 ausgewertet und einem Rechner 20 zugeführt. In diesem Rechner 20 erfolgt zunächst eine Vorverarbeitung der pulsatilen Anteile der Lichtintensitäten $I_{ind\ puls}(t)$ und $I_{ref\ puls}(t)$, beispielsweise durch Quotientenbildung:

$$I_{ind\ puls}(t) = I_{ind}(t-dt) / I_{ind}(t+dt)$$

$$I_{ref\ puls}(t) = I_{ref}(t-dt) / I_{ref}(t+dt)$$

Aus diesen vorverarbeiteten Signalen kann in einem weiteren Schritt bereits die relative Farbstoff-Konzentration als Funktion der Zeit bestimmt werden, und zwar ebenfalls durch Quotientenbildung:

$$c(t) = I_{ind\ puls} / I_{ref\ puls}$$

Es bedarf hier keines Kalibrationsfaktors, da für die Bestimmung der Leberfunktion nur ein lineares Signal benötigt wird. Das Meßprinzip beruht nicht auf der Bestimmung absoluter Konzentrationswerte, sondern nur auf der Bestimmung von Zeitkonstanten.

Bei der weiteren Auswertung der Farbstoff-Konzentration als Funktion der Zeit wird anschließend mittels nichtlinearer Anpassungsalgorithmen aus c(t) eine Kreislauftransportfunktion g(t), die das Transportverhalten des Kreislaufs beschreibt, sowie ein Parameter k ermittelt, der eine fraktionelle Wiedereintrittsrate des Farbstoffes nach jeder Kreislaufpassage darstellt.

Im folgenden legt man ein Modell zugrunde, bei dem ein Transportweg des injizierten Indikatorfarbstoffes ICG als Bolusinjektion zuerst durch die Lunge führt. Hinter der Lunge teilt sich der Transportweg in ein erstes, schnelles Kompartiment, ein zweites langsames Kompartiment und in eine Elimination des Farbstoffes durch die Leber. Das erste und zweite Kompartiment führen anschließend wieder zur Lunge zurück. Die Konzentration des Indikatorfarbstoffes am Ausgang der Lunge folgt der Funktion:

$$c(t) = c_{bolus}(t) + c_{rez}(t)$$

mit

$$c_{rez}(t) = k * \int_0^t g(t-u) * c(u)\ du \ .$$

Hierbei bilden c(t) den Konzentrations-Zeit-Verlauf des Indikatorfarbstoffes, $c_{bolus}(t)$ der den Meßort direkt passierende erste Anteil des Farbstoff-Konzentrations-Zeit-Verlaufs, $c_{rez}(t)$ einen rezirkulierender Anteil des Farbstoff-Konzentrations-Zeit-Verlaufs und k die fraktionelle Wiedereintrittsrate des Farbstoffes nach teilweiser Eliminierung durch die Leber. Der Faktor k ist daher stets kleiner als 1.

Der Transportprozeß wird durch ein Faltungintegral beschrieben. Rezirkulation in dieser Darstellungsweise bedeutet, daß das Ergebnis der Faltung $c_{rez}(t)$ gleichzeitig auch die Eingangsfunktion c(t) beeinflußt. Die Rezirkulation des Indikators führt somit zu einem Zusammenhang, der im Prinzip folgendermaßen beschrieben ist:

$$c(t) = f[k, g(t), c(t)]$$

Für g(t) werden die Kompartimente des Kreislaufmodells angesetzt. Voruntersuchungen ergaben, daß bei Farbstoffverdünnungskurven, die an Patienten gemessen worden sind, für g(t) ein bis zwei Kompartimente anzusetzen sind, je nachdem, welche Genauigkeit angestrebt wird. Damit ergibt sich für die allgemeine Modellfunktion

$$g(t) = a_m g_m(t) + a_{m+1} g_{m+1}(t) + ... + a_n g_n(t)$$

mit

$$\sum_{m=1}^{n} a_m = 1,$$

bei der die einzelnen Kompartimente $a_m g_m$ durch linksschiefe Verteilungsfunktionen beschrieben sind. Für zwei Kompartimente gilt der spezielle Ausdruck:

$$g(t) = a_1 g_1(t) + a_2 g_2(t).$$

Die Berechnung der Kreislauftransportfunktionen aus gemessenen Farbstoffkurven wird durch den Rechner mit Hilfe eines iterativen, nicht-linearen Anpassungsverfahrens durchgeführt werden, bei dem unter Vorgabe der Modellfunktion

$$g(t) = a_1 g_1(t) + a_2 g_2(t)$$

wiederholt eine rekursive Faltung gemäß den Gleichungen

$$c(t) = c_{bolus}(t) + c_{rez}(t)$$

und

$$c_{rez}(t) = k * \int_0^t g(t-u) * c(u) \, du$$

durchgeführt wird, wobei k, $a_1$, $a_2$ und die Parameter der Verteilungsfunktionen nach dem Verfahren der kleinsten quadratischen Abweichung optimiert werden.

Nach Durchführung dieser Rechenschritte ergibt sich eine Transportfunktion, wie sie als Beispiel für die in Fig. 1 enthaltenen Daten für zwei Kompartimente errechnet wurde und in Fig. 3 dargestellt ist. Die resultierende Transportfunktion g(t) setzt sich aus der Transportfunktion g1(t) für das erste Kompartiment und g2(t) für das zweite Kompartiment zusammem.

Aus der Transportfunktion g(t) wird des weiteren die mittlere Kreislauftransitzeit berechnet ($mtt_{circ}$), die das erste Moment von g(t) ist:

$$mtt_{circ} = \frac{\int_0^\infty g(t) \bullet t \, dt}{\int_0^\infty g(t) \, dt}$$

Die Plasmaverschwinderate (PDR) des Farbstoffes ICG ergibt sich sodann als:

EP 1 022 984 B1

$$PDR = (1-k)/(k \cdot mtt_{circ})$$

**Patentansprüche**

1. Vorrichtung zur Bestimmung der Leberfunktion anhand einer Plasmaverschwindungrate (PDR) mit einem optischen Meßsensor (10) und einem damit verbundenen Rechner (12), wobei mittels des Meßsensors (12) nach Injektion eines Indikatorfarbstoffes in den Blutkreislauf die im Blutkreislauf resultierende Farbstoffkonzentration gemessen wird und mittels des Rechners (12) die Plasmaverschwindungrate aus dem Farbstoff-Konzentrations-Zeitverlauf des Indikatorfarbstoffes bestimmbar ist, **dadurch gekennzeichnet, daß** der Rechner (12) so gesteuert ist, daß aus der Messung der Farbstoffkonzentration (c(t)) eine mittlere Kreislauftransitzeit ($mtt_{circ}$) berechnet wird, daß ein Parameter (k) ermittelt wird, der eine fraktionelle Wiedereintrittsrate des Indikatorfarbstoffes nach jeder Kreislaufpassage darstellt, und daß die Plasmaverschwindungrate (PDR) nach der Formel

$$PDR = (1-k)/(k \cdot mtt_{circ})$$

berechnet wird.

2. Vorrichtung nach Anspruch 1, **dadurch gekennzeichnet, daß** der Rechner (12) ferner so gesteuert ist, daß die mittlere Kreislauftransitzeit ($mtt_{circ}$) aus einer Kreislauftransportfunktion (g(t))berechnet wird, die das Transportverhalten des Kreislaufs beschreibt, nach der Formel

$$mtt_{circ} = \frac{\int_0^\infty g(t) \bullet t\, dt}{\int_0^\infty g(t)\, dt}$$

3. Vorrichtung nach Anspruch 2, **dadurch gekennzeichnet, daß** der Rechner (12) so gesteuert ist, daß die Berechnung der Kreislauftransportfunktion (g(t)) aus der gemessenen Farbstoffkonzentration mit Hilfe eines iterativen, nichtlinearen Anpassungsverfahrens durchgeführt wird, bei dem unter Vorgabe einer Modellfunktion

$$g(t) = a_m g_m(t) + a_{m+1} g_{m+1}(t) + ... + a_n g_n(t)$$

mit

$$\sum_{m=1}^{n} a_m = 1,$$

bei der die einzelnen Kompartimente $a_m g_m$ durch linksschiefe Verteilungsfunktionen beschrieben sind, wiederholt eine rekursive Faltung gemäß den Gleichungen

$$c(t) = c_{bolus}(t) + c_{rez}(t)$$

und

$$c_{rez}(t) = k * \int_0^t g(t-u) * c(u) \, du$$

durchgeführt wird und die Parameter k, $a_m$ sowie die Parameter der Verteilungsfunktionen nach dem Verfahren der kleinsten quadratischen Abweichung optimiert werden, wobei wenigstens ein Kompartiment $a_1g_1(t)$ angesetzt wird und c(t) den Konzentrations-Zeit-Verlauf des Indikatorfarbstoffes, $c_{bolus}(t)$ der den Meßort direkt passierende erste Anteil des Farbstoff-Konzentrations-Zeit-Verlaufs, $c_{rez}(t)$ einen rezirkulierender Anteil des Farbstoff-Konzentrations-Zeit-Verlaufs und k die Eliminationsfraktion des durch die Leber eliminierten Farbstoffes bilden.

4. Vorrichtung nach Anspruch 3, **dadurch gekennzeichnet, daß** der Rechner (12) so gesteuert ist, daß zwei Kompartimente $(a_1g_1(t))$ und $(a_2g_2(t))$ angesetzt werden.

5. Vorrichtung nach einem der Ansprüche 1 bis 4, **dadurch gekennzeichnet, daß** der optische Meßsensor (10) ein fiberoptischer Katheter ist, der zur Messung in einem zentralen Gefäß angeordnet wird.

6. Vorrichtung nach einem der Ansprüche 1 bis 4, **dadurch gekennzeichnet, daß** der optische Meßsensor (10) einen Lichtsender und Lichtempfänger zur nicht-invasiven Licht-Transmissions- oder Reflexionsmessung eingestrahlten Lichtes an geeigneten Körperorten, insbesondere am Finger, Ohrläppchen, Nasenrücken, Wangenschleimhaut oder Stirn umfaßt.

**Claims**

1. A device for measuring liver function on the basis of a plasma disappearance rate (PDR) having an optical measuring sensor (10) and a computer (12) connected thereto, wherein, following injection of an indicator dye into the bloodstream, the resultant dye concentration in the bloodstream is measured using the measuring sensor (12) and the plasma disappearance rate can be determined from the dye-concentration time course of the indicator dye using the computer (12), **characterised in that** the computer (12) is operated in such a way that a mean circulation transit time $(mtt_{circ})$ is calculated from the measurement of the dye concentration (c(t)), a parameter (k) representing a fractional re-entry rate of the indicator dye following each passage through the circulation is determined, and the plasma disappearance rate (PDR) is calculated according to the formula

$$PDR = (1-k) / (k \cdot mtt_{circ}).$$

2. A device according to Claim 1, **characterised in that** the computer (12) is furthermore operated in such a way that the mean circulation transit time $(mtt_{circ})$ is calculated from a circulation transport function (g(t)), which describes the transport behaviour of the circulation, according to the formula

$$\frac{\int_0^\infty g(t) \cdot t \, dt}{\int_0^\infty g(t) \, dt}$$

3. A device according to Claim 2, **characterised in that** the computer (12) is operated in such a way that the circulation transport function (g(t)) is calculated from the measured dye concentration with the aid of an iterative, non-linear matching process wherein, assuming a model function

$$g(t) = a_m g_m(t) + a_{m+1} g_{m+1}(t) + ... + a_n g_n(t)$$

where $\sum_{m=1}^{n} a_m = 1$,

in which the individual compartments $a_m g_m$ are described by left-skewed distribution functions, recursive convolution is repeatedly performed according to the equations

$$c(t) = c_{bolus}(t) + c_{rec}(t)$$

and

$$c_{rec}(t) = k * \int_{0}^{t} g(t-u) * c(u) \, du,$$

and the parameters k, $a_m$ and distribution-function parameters are optimised by the least-square deviation method , wherein at least one compartment $a_1 g_1(t)$ is specified and c (t) represents the concentration-time course of the indicator dye, $c_{bolus}(t)$ the concentration-time course of the first fraction of the dye directly passing the measuring site, $c_{rec}(t)$ the concentration-time course of a recirculating fraction of the dye, and k the elimination fraction of the dye eliminated by the liver.

4. A device according to Claim 3, **characterised in that** the computer (12) is operated in such a way that two compartments ($a_1 g_1(t)$) and ($a_2 g_2(t)$) are specified.

5. A device according to one of Claims 1 to 4, **characterised in that** the optical measuring sensor (10) is a fibre-optic catheter which, for measuring purposes, is positioned in a central vessel.

6. A device according to one of Claims 1 to 4, **characterised in that** the optical measuring sensor (10) comprises a light transmitter and light receiver for non-invasive light-transmission or light-reflection measurement of irradiated light at suitable body sites, in particular the finger, ear lobe, bridge of the nose, buccal mucosa or forehead.


**Revendications**

1. Dispositif de définition de la fonction du foie au moyen d'un taux de disparition de plasma (PDR) avec un capteur de mesure optique (10) et un calculateur qui y est relié (12), dans lequel on mesure, au moyen du capteur de mesure (10) après injection d'un colorant indicateur dans la circulation sanguine, la concentration en colorant résultante dans la circulation sanguine et on peut définir, au moyen du calculateur (12), le taux de disparition de plasma à partir de la durée de la concentration en colorant du colorant indicateur,
   **caractérisé en ce que**
   le calculateur (12) est commandé de telle sorte que, à partir de la mesure de la concentration en colorant (c(t)), une vitesse circulatoire transitoire moyenne ($mtt_{circ}$) est calculée, **en ce qu'**un paramètre (k) représentant un taux de réentrée fractionnelle du colorant indicateur est déterminé après chaque passage circulatoire et en **en ce que** le taux de disparition de plasma (PDR) est défini par la formule

$$PDR = (1-k) / (k \cdot mtt_{circ})$$

2. Dispositif selon la revendication 1, **caractérisé en ce que**
   le calculateur (12) est en outre commandé de telle sorte que la vitesse circulatoire transitoire moyenne ($mtt_{circ}$) est calculée à partir d'une fonction de transport circulatoire (g(t)) qui représente le comportement au transport de la circulation, à partir de la formule :

$$mtt_{circ} = \frac{\int_0^\infty g(t) \cdot t \, dt}{\int_0^\infty g(t) \, dt}$$

**3.** Dispositif selon la revendication 2, <u>**caractérisé en ce que**</u>
le calculateur (12) est commandé de telle sorte que le calcul de la fonction de transport circulatoire (g(t)) est effectué à partir de la concentration en colorant mesurée à l'aide d'une procédure d'ajustement itératif non-linéaire, dans lequel en prescrivant une fonction type

$$g(t) = a_m g_m(t) + a_{m+1} g_{m+1}(t) + \dots a_n g_n(t)$$

avec

$$\sum_{m=1}^{n} a_m = 1$$

dans laquelle les compartiments individuels $a_m g_m$ sont représentés par des fonctions de distribution négativement asymétriques, une convolution récursive est répétée selon les équations

$$c(t) = c_{bolus}(t) + c_{rez}(t)$$

et

$$C_{rez}(t) = k * \int_o^t g(t-u) * c(u) \, du$$

et les paramètres k, $a_m$ ainsi que les paramètres des fonctions de distribution sont optimisés selon le procédé du plus petit écart quadratique, où au moins un compartiment $a_1 g_1(t)$ est fixé et c(t) représente la courbe temporelle de concentration en colorant indicateur, $c_{bolus}(t)$ représente la première partie de la courbe temporelle de concentration en colorant passant directement par le lieu de mesure, $c_{rez}(t)$ représente une partie recirculante de la courbe temporelle de concentration en colorant et k représente la fraction d'élimination du colorant éliminé par le foie.

**4.** Dispositif selon la revendication 3, <u>**caractérisé en ce que**</u>
le calculateur (12) est commandé de telle sorte que deux compartiments ($a_1 g_1(t)$) et ($a_2 g_2(t)$) sont fixés.

**5.** Dispositif selon l'une des revendications 1 à 4, <u>**caractérisé en ce que**</u>
le capteur de mesure optique (10) est un cathéter en fibre optique qui est disposé dans un récipient central pour la mesure.

**6.** Dispositif selon l'une des revendications 1 à 4, <u>**caractérisé en ce que**</u>
le capteur de mesure optique (10) comprend un émetteur de lumière et un récepteur de lumière pour mesurer la réflexion ou la transmission de lumière non-invasive de la lumière émise en des emplacements appropriés du corps, en particulier au niveau des doigts, des lobes d'oreilles, du dos du nez, de la muqueuse des joues ou du front.

Fig. 1

Fig. 2

$g_1(t)$  $g_2(t)$

0    30    60    90    120    150

Fig. 3

Fig. 4